# EUROPÄISCHE PATENTSCHRIFT

(11) **EP 3 063 266 B1**
(45) Veröffentlichungstag und Bekanntmachung des Hinweises auf die Patenterteilung: **28.10.2020**
(21) Anmeldenummer: 14772288.8
(22) Anmeldetag: 22.09.2014
(51) Int. Cl.: C12N 5/00

(54) **VERFAHREN ZUR KULTIVIERUNG VON ZELLEN IN ADHÄSIONSKULTUR UNTER VERWENDUNG EINES ZELLKULTUR-TRÄGERS IN KAPSELFORM, SOWIE ZELLKULTUR-TRÄGER DAFÜR**
METHOD FOR CULTIVATING CELLS IN ADHESION CULTURE BY USING A CELL CULTURE CARRIER IN CAPSULE FORM, AND CELL CULTURE CARRIER THEREFOR
PROCÉDÉ POUR LA CULTURE DE CELLULES DANS UNE CULTURE SELON LA MÉTHODE DE LA GOUTTE PENDANTE EN UTILISANT UN SUPPORT POUR CULTURE CELLULAIRE SOUS FORME DE CAPSULE ET SUPPORT DE CULTURE POUR CE PROCÉDÉ

(30) Priorität: 30.10.2013 DE 102013018242
(43) Veröffentlichungstag der Anmeldung: 07.09.2016
(73) Patentinhaber: Fraunhofer-Gesellschaft zur Förderung der angewandten Forschung e.V., 80686 München (DE)
(72) Erfinder: RAPOPORT, Daniel Hans, 23564 Lübeck (DE); VOIGT, Miriam, 23611 Bad Schwartau (DE); WALTER, Charlotte, 23562 Lübeck (DE)
(74) Vertreter: v. Bezold & Partner Patentanwälte - PartG mbB
(86) Internationale Anmeldenummer: PCT/EP2014/002567
(87) Internationale Veröffentlichungsnummer: WO 2015/062686

(56) Entgegenhaltungen:
- WO-A1-2005/121319
- WO-A2-2007/146319
- YANG LIU ET AL: "Production of endothelial cell-enclosing alginate-based hydrogel fibers with a cell adhesive surface through simultaneous cross-linking by horseradish peroxidase-catalyzed reaction in a hydrodynamic spinning process", JOURNAL OF BIOSCIENCE AND BIOENGINEERING, Bd. 114, Nr. 3, 24. April 2012 (2012-04-24), Seiten 353-359, XP028411225, ELSEVIER, AMSTERDAM, NL ISSN: 1389-1723, DOI: 10.1016/J.JBIOSC.2012.04.018 [gefunden am 2012-05-02]
- FRIEDERIKE EHRHART ET AL: "Biocompatible Coating of Encapsulated Cells Using Ionotropic Gelation", PLOS ONE, Bd. 8, Nr. 9, 9. September 2013 (2013-09-09), Seite e73498, XP055147125, DOI: 10.1371/journal.pone.0073498
- MOON J S ET AL: "Morphology and metabolism of hepatocytes microencapsulated with acrylic terpolymer-alginate using gelatin and poly(vinyl alcohol) as extracellular matrices", JOURNAL OF BIOMATERIALS SCIENCE. POLYMER EDITION, Bd. 16, Nr. 10, 1. Januar 2005 (2005-01-01), Seiten 1245-1259, XP009180802, VSP, UTRECHT, NL ISSN: 0920-5063
- SAKAI S ET AL: "Calcium alginate microcapsules with spherical liquid cores templated by gelatin microparticles for mass production of multicellular spheroids", ACTA BIOMATERIALIA, Bd. 6, Nr. 8, 1. August 2010 (2010-08-01), Seiten 3132-3137, XP027103070, ELSEVIER, AMSTERDAM, NL ISSN: 1742-7061 [gefunden am 2010-02-08]
- YAO RUI ET AL: "Alginate and alginate/gelatin microspheres for human adipose-derived stem cell encapsulation and differentiation.", BIOFABRICATION, Bd. 4, Nr. 2, 025007, Juni 2012 (2012-06), Seiten 1-11, XP002731267, ISSN: 1758-5090, DOI: 10.1088/1758-5082/4/2/025007
- ASHIDA TOMOAKI ET AL: "Competing two enzymatic reactions realizing one-step preparation of cell-enclosing duplex microcapsules.", BIOTECHNOLOGY PROGRESS, Bd. 29, Nr. 6, 1. September 2013 (2013-09-01), Seiten 1528-1534, XP002731268, ISSN: 1520-6033

## Beschreibung

### Hintergrund der Erfindung

Die effiziente Vermehrung adhärent wachsender Zellen stellt nach wie vor ein ungelöstes Problem dar, obwohl diesbezüglich eine Reihe unterschiedlicher Ansätze verfolgt wurden (hinsichtlich eines Überblicks siehe z.B. Pörtner et al. in Journal of Bioscience and Bioengineering, Bd. 100, Nr. 3, 235-245, (2005) und Warnock et al. in Biotechnol. Appl. Biochem., Bd. 45, 1-12 (2006)).

Mit den derzeit besten technischen Vorrichtungen (Festbettreaktoren, Hohlfaserreaktoren) können lediglich Vermehrungsfaktoren von ca. 10², bezogen auf einen Batch-Vorgang, in der Zellkulturtechnik auch als "Passage" bezeichnet, erreicht werden. Wünschenswert wären jedoch deutlich höhere Faktoren von 10³ (z.B. für die regenerative Medizin und Zellbanken, 10⁴ (z.B. Produktionszellen für Biomoleküle) bis 10⁶ (z.B. für die Ernährungs- und Landwirtschaft).

Dem steht entgegen, dass adhärente Zellen nur in einem engen Dichtebereich proliferativ sind. (Flächendichte ca. 500-50.000 Zellen/cm²). Außerhalb dieses Dichtebereichs wachsen die Zellen nicht oder sterben ab.

Abhilfe könnte eine mit der Zellzahl sich vergrößernde Oberfläche schaffen. Die mit diesem Ansatz verbundenen Probleme sind jedoch technisch bislang noch nicht gelöst. Die liegt zum einen daran, dass die Oberflächenvergrößerung einem exponentiellen Wachstum folgen müsste, zum anderen daran, dass die technischen Möglichkeiten zur Realisierung wachsender Oberflächen bisher sehr beschränkt sind.

Die derzeit beste Möglichkeit besteht in der Verwendung von Mikrocarriern. Das sind partikelförmige Körper (häufig sphärisch geformt, aber auch Plättchen- und Stäbchenform möglich) von typischerweise ca. 1 mm Durchmesser, auf deren Oberfläche die Zellen wachsen können. Die Nährstoffzufuhr geschieht durch ein Medium, in dem die Mikrocarrier suspendiert werden. Sobald die Zellen den Carrier vollständig bewachsen haben, werden sie (in der Regel enzymatisch) abgelöst und auf neue Carrier gesät. Mit der Zahl der Carrier steigt auch die Wachstumsoberfläche.

Dieser Ansatz hat eine Reihe von Nachteilen. Erstens muss die Reaktorgröße der Zahl der Carrier angepasst sein; der Volumenanteil der Carrier am Gesamtreaktionsvolumen muss sich in gewissen Grenzen bewegen (in der Regel 25-50%). Dadurch verbietet sich die Verwendung ein und desselben Reaktors zur Erzielung hoher Expansionsfaktoren; stattdessen muss von kleineren zu größeren Reaktoren fortgeschritten werden. Zweitens ist stets eine Prozesstechnik erforderlich, die mechanisch schonend mit Festkörpern (den Mikrocarriern) umgehen kann, was apparativ wesentlich aufwändiger ist, als der Umgang mit Flüssigkeiten. Flüssigkeiten können sehr viel einfacher gefördert, filtriert, sterilisiert etc. werden. Drittens ist die Volumennutzung des Reaktors nicht sehr effizient, weil die Zellen lediglich an der Oberfläche der Carrier wachsen können. Viertens sind die Zellen auf den Mikrocarriern Scher- und Prallkräften ausgesetzt, die durch Konvektion des Mediums und Aufeinanderprallen der Carrier entstehen. Fünftens ist die Analyse der Zellen auf den Mikrocarriern schwierig. In der Regel lassen sich die Zellen auf den Carriern schlecht mikroskopieren. Sechstens geschieht die Versorgung der Zellen in der Regel auschließlich durch das Medium, das volumenfüllend und möglichst gradientenfrei bereitgestellt werden muss. Dies bedingt eine ineffiziente Nutzung (Utilisation) des Mediums. Siebtens stellen sowohl die Aussaat der Zellen auf die Carrier (Inokulation) als auch die Ernte der Zellen von den Carriern technisch schwierige Prozesse dar, bei denen große Anteile der Zellen verloren gehen.

Vor diesem Hintergrund besteht eine Aufgabe der Erfindung in der Bereitstellung von verbesserten Mitteln und Verfahren zur effizienten Kultivierung von Zellen in Adhäsionskultur, mit denen die geschilderten Nachteile des Standes der Technik vermieden oder wenigstens stark verringert werden können.

Diese Aufgabe konnte durch die Bereitstellung des Kultivierungsverfahrens nach Anspruch 1 gelöst werden. Speziellere Aspekte und bevorzugte Ausführungsformen der Erfindung sind Gegenstand der weiteren Ansprüche.

### Beschreibung der Erfindung

Das erfindungsgemäße Verfahren zur Kultivierung von Zellen in Adhäsionskultur umfasst mindestens die folgenden Schritte:
a) Lösen oder Suspendieren eines vernetzungsfähigen, biokompatiblen Materials mit Adhäsionsstellen für Zellen in einem Zellkulturmedium,
b) Suspendieren von Zellen in dem Zellkulturmedium, welches das vernetzungsfähige, biokompatible Material enthält, oder in einem Medium, das mindestens eine Komponente enthält, welche zur Vernetzung des vernetzungsfähigen, biokompatiblen Materials erforderlich ist,
c) Eintropfen der Zellsuspension in ein Medium unter Bedingungen, welche die Vernetzung des biokompatiblen Materials initiieren oder erlauben, wobei entweder die Zellsuspension oder das Medium, in welches die Zellsuspension eingetropft wird, das vernetzungsfähige biokompatible Material enthält,
d) Bilden von stabilen, vorzugsweise porösen, Kapseln aus vernetztem biokompatiblem Material, welche inkorporierte adhärente Zellen enthalten,
e) Proliferieren der adhärenten Zellen in den Kapseln für eine vorgegebene Zeitspanne,
f) Auflösen des Kapselmaterials durch einen physikalischen oder chemischen Stimulus und Freisetzen der Zellen als Zellsuspension,
und ist dadurch gekennzeichnet, dass die in Schritt f) freigesetzten Zellen erneut in einem Zellkulturmedium, welches ein vernetzungsfähiges, biokompatibles Material enthält, oder in einem Medium, das mindestens eine Komponente enthält, welche zur Vernetzung des vernetzungsfähigen, biokompatiblen Materials erforderlich ist, suspendiert und die Schritte c)-f) mindestens einmal wiederholt werden.

Auf diese Weise lässt sich auf einfache und effiziente Weise eine erhebliche Ausbeutesteigerung erzielen.

Der Begriff "vernetzungsfähiges biokompatibles Material", wie hier verwendet, soll erstens ausdrücken, dass das betreffende Material einer Polymerisations-, Additions-, Kondensations- oder anderen Verknüpfungsreaktion zugänglich ist, die zu einem makromolekularen Produkt, vorzugsweise einem Hydrogel, führt, und zweitens, dass dieses Material und das resultierende makromolekulare Produkt für die Zellen im Wesentlichen nicht toxisch ist.

Als Zellen zur Kultivierung in den erfindungsgemäß verwendeten Zellkultur-Trägern kommen grundsätzlich alle Zellen in Betracht, die in Adhäsionskultur vermehrt werden können, konkreter alle obligat adhärent wachsenden Zellen. Spezielle, nicht-beschränkende Beispiele für solche Zellen umfassen Stammzellen, somatische Zellen, Primärzellen, genetisch veränderte Zellen (e.g. ips-Zellen) und adhärent wachsende Produktionszelllinien (e.g. CHO-Zellen) sowie Tumorzellen etc..

Die Proliferation der Zellen in Schritt e) geschieht typischerweise für eine Zeitspanne im Bereich von einigen Stunden bis 50 Tagen, beispielsweise 3-10 Tagen. Diese Zeitspanne ist jedoch nicht kritisch und es kann gewünschtenfalls, z.B. für bestimmte Zellarten, auch davon abgewichen werden. Meist wird Schritt e) solange durchgeführt werden, bis die Aufnahmekapazität der Kapseln erschöpft ist.

Im erfindungsgemäßen Verfahren umfassen die Bedingungen, welche die Vernetzung des biokompatiblen Materials in Schritt c) initiieren oder erlauben, die Exposition des biokompatiblen Materials gegenüber einem physikalischen oder chemischen Stimulus.

Dieser physikalische oder chemische Stimulus in Schritt c) ist aus der Gruppe, die eine Temperaturänderung, Druckänderung, Ultraschall, elektromagnetische Strahlung, insbesondere sichtbares Licht und UV, ein pH-Änderung, die Einwirkung von Enzymen, Radikalstartern, Wasserzugabe oder Wasserausschluss, Ionen, insbesondere Kationen, umfasst, ausgewählt.

In spezielleren Ausführungsformen ist der physikalische Stimulus in Schritt c) beispielsweise eine Ultraschallbehandlung des biokompatiblen Materials und der chemische Stimulus in Schritt c) ist beispielsweise der Kontakt des biokompatiblen Materials mit einem chemischen Agens, insbesondere ausgewählt aus der Gruppe, die aus einem Enzym, z.B. Thrombin, und einem 2-wertiges Kation, z.B. Ca²⁺ oder Ba²⁺, besteht oder diese umfasst.

In einer noch spezielleren Ausführungsform des erfindungsgemäßen Verfahrens wird entweder eine Zellsuspension, die Alginat bzw. Alginat/Gelatine als vernetzungsfähiges biokompatibles Material enthält, in ein Medium, das ein zweiwertiges Kation, z.B. Ca²⁺ oder Ba²⁺, enthält, getropft, oder eine Zellsuspension in einem Medium, das ein zweiwertiges Kation, z.B. Ca²⁺ oder Ba²⁺, enthält, wird zu einem Medium, das Alginat bzw. Alginat/Gelatine als vernetzungsfähiges biokompatibles Material enthält, getropft. Bei der letzteren Verfahrensvariante entstehen kleine Flüssigkeitsräume, die von einer Gelmembran umgeben sind. In diesen Räumen können die Zellen ebenfalls erfolgreich proliferieren.

Im erfindungsgemäßen Verfahren ist der Stimulus in Schritt f) aus der Gruppe ausgewählt, welche eine Temperaturänderung, Druckänderung, Ultraschall, elektromagnetische Strahlung, insbesondere sichtbares Licht und UV, ein pH-Änderung, die Einwirkung von Enzymen, insbesondere Proteasen und zuckerspaltenden Enzymen, Komplexbildnern oder Lösungsmitteln, oder Kombinationen davon umfasst.

Spezieller stellt der Stimulus in Schritt f) die Einwirkung von Proteasen, die von den adhärenten Zellen selbst produziert werden, dar oder umfasst eine solche Einwirkung.

In einer bevorzugten Ausführungsform der Erfindung ist das Kapselmaterial porös. Die Poren haben typischerweise eine Größe im Bereich von 50 bis 3000 µm, vorzugsweise im Bereich von 100 bis 1000 µm.

In einer Ausführungsform des erfindungsgemäßen Verfahrens wird Schritt c) daher unter Bedingungen durchgeführt, welche die Porenbildung im Kapselmaterial fördern.

Dies kann beispielsweise dadurch geschehen, dass in das Medium, in welches die Zellsuspension eingetropft wird, ein Gas, beispielsweise Luft, eingepresst wird. Während oder nach erfolgter Bildung des Kapselmaterials, typischerweise ein Gel, entweicht das Gas langsam wieder und erzeugt dabei Poren.

In einer anderen Variante wird ein gasbildendes partikuläres Material, z.B. ein Carbonat, welches nach Exposition gegenüber einem physikalischen oder chemischen Stimulus, z.B. Säure, vorzugsweise eine milde Säure wie Essigsäure etc., Gas freisetzt, in das Kapselmaterial eingeschlossen. Das partikuläre Material wird dabei typischerweise in Form eines unlöslichen feinen Pulvers (Teilchengröße im Bereich weniger Mikrometer, z.B. 1-500, 1-100, 1-50 oder 1-10 µm) zugegeben.

Das erfindungsgemäße Verfahren ist ferner dadurch gekennzeichnet, dass das vernetzungsfähige biokompatible Material ein Material darstellt oder umfasst, welches ein Hydrogel bilden kann.

Dieses Hydrogel kann ein auf Naturstoffen basierendes Hydrogel, z.B. ein protein-basiertes Hydrogel, ein zucker-basiertes Hydrogel, enthaltend modifizierte Oligo- oder Polysaccharide, oder ein synthetisches Hydrogel auf der Basis von Polyestern, Polyethern oder Polyalkoholen sein und/oder ein thermoresponsives Material, z.B. Poly-NIPAM oder Poly(hydroxypropylcellulose), umfassen.

In einer bevorzugten Ausführungsform des erfindungsgemäßen Verfahrens wird das vernetzungsfähige, biokompatible Material durch eine chemische Kopplungsreaktion mit Adhäsionsstellen für Zellen versehen.

Diese Adhäsionsstellen für Zellen können beispielsweise spezifische oder unspezifische Adhäsions- oder Bindungsmotive für biologische Zellen, z.B. Aminogruppen, RGD-Peptide, RAD-Peptide oder Analoga davon, darstellen oder umfassen.

In spezielleren Ausführungsformen des erfindungsgemäßen Verfahrens umfassen die Kapseln aus vernetztem biokompatiblen Material ein thermoresponsives Material, z.B. Poly-NIPAM oder Poly(hydroxypropylcellulose), einen Polyester, Polyether oder Polyalkohol, Seide, Fibrin oder vernetztes Alginat.

Seide kann per Ultraschall vernetzt werden (Konformations-änderung, Bildung semikristalliner Bereiche, Wasserausschluss).

Fibrin entsteht durch Vernetzung von Fibrinogen mit Thrombin/- Calcium. Dabei wird das Fibrinogen proteolytisch gespalten und in ein aktives Monomer überführt, das mit weiteren Monomeren reagieren kann und so das Fibrin-Gel bildet.

Alginat und davon abgeleitete Hydrogele können durch Vernetzung mit zweiwertigen Ionen, insbesondere Ca²⁺ oder Ba²⁺, gebildet werden.

Polyester können durch Einstellung eines günstigen (sauren, aber zellphysiologisch tolerablen) pH-Wertes aus den Monomeren gebildet werden; es können prinzipiell sowohl synthetische als auch natürliche Polyester verwendet werden.

Polyalkohole bzw. Polyole können durch Umesterung von Glycerin-Fetten hergestellt werden. Falls die dabei verwendeten Reaktionsbedingungen zellphysiologisch nicht tolerabel sind, können auch die fertigen Polymere eingesetzt werden, besonders in Form schaltbarer Block-Polymere. Diese können auf Temperatur- oder pH-Unterschiede mit Wasserausschluss reagieren und auf diese Weise vernetzen. Gleiches gilt für Poly-NIPAM und Polyhydroxypropylcellulose.

In einer noch spezielleren Ausführungsform ist das Verfahren dadurch gekennzeichnet, dass an eine Alginat-Gerüststruktur Gelatinemoleküle angekoppelt wurden, vorzugsweise mittels einer Carbodiimid-Kopplungsreaktion, und die Vernetzung dieses Alginat-Gelatine-Vorläufermaterials durch Kontaktierung mit einem zweiwertigen Kation, insbesondere Ca²⁺ oder Ba²⁺, erfolgt.

Die Herkunft und Molmasse der verwendeten Gelatine ist nicht kritisch. Es kann sowohl Gelatine, die mit einem sauren Hydrolyseverfahren hergestellt wurde, als auch Gelatine mit einem alkalischen Herstellungsverfahren verwendet werden.

Die Molmasse kann grundsätzlich in einem weiten Bereich von beispielsweise 1000 bis 100.000 g/mol variieren. Eine geeignete Molmasse kann z.B. in einem typischen Bereich für kommerzielle Gelatinesorten, d.h. etwa 50.000-80.000 g/mol, liegen.

Jedoch ist auch ein Gelatinehydrolysat mit einer wesentlich kleineren Molmasse, z.B. ca. 1000-10.000, spezieller 3000 bis 5000 g/mol, gut einsetzbar. Gelatine mit solchen kleineren Molmassen kann beispielsweise durch weiteren enzymatischen Abbau kommerzieller Produkte erhalten werden.

Der Vorteil dieser Produkte mit kleinerer Molmasse liegt darin, dass sie in höherer Monomer-Konzentration gelöst werden können, ohne dabei gelierend zu wirken und die Kopplungsreaktion zu behindern. Bei Gelatine mit einer Molmasse von z.B. etwa 50.000-80.000 g/mol, insbesondere im oberen Bereich, kann ab Konzentrationen von etwa 1 % im Reaktionsmedium und bei tiefen Temperaturen die Viskosität so hoch werden, dass das Rühren sehr erschwert wird.

Einige nicht-beschränkende Bespiele für geeignete kommerzielle Gelatinesorten sind im Folgenden aufgeführt. Die Gelatine ist dabei durch ihre Gelierkraft gekennzeichnet, die in Bloom gemessen wird. Die Einheit Bloom gibt die Masse eines Prüfstempels an, der einen genormtem Eindruck in die Gelatine pressen kann.
a) Gelatine Typ A (saures Verfahren), 225 Bloom, Fa. Hahn
b) Gelatine Typ A, 300 Bloom, Schweinehaut, Fa. Sigma
c) Gelatine Typ A, 90-110 Bloom, Schweinehaut, Fa. Sigma
d) Gelatine Typ B (alkalisches Verfahren), Schweinehaut, Fa. Sigma

Die Herkunft und Molmasse des verwendeten Alginats ist nicht besonders beschränkt. In einer speziellen Ausführungsform wurde Natriumalginat aus Braunalgen (von der Fa. BioReagent im Reinheitsgrad "Cell culture tested", d.h. hinreichend toxinfrei und steril, bezogen) verwendet. Das eingesetzte Alginat besaß "low viscosity", befand sich also im unteren Molmassenbereich zwischen 50.000 und 80.000 g/mol.

In einer bevorzugten Ausführungsform des erfindungsgemäßen Verfahrens enthalten die Kapseln aus vernetztem biokompatiblen Material ferner immobilisierte Nährstoffe und/oder Wachstumshormone. Auf diese Weise kann das Wachstum der Zellen mit relativ geringen Mengen an Wirkstoffen sehr effizient gefördert werden.

In einer besonders bevorzugten Ausführungsform des erfindungsgemäßen Verfahrens enthalten die Kapseln aus vernetztem biokompatiblen Material ferner immobilisierte Reportermoleküle. Mit Hilfe solcher Reportermoleküle können beispielweise die Wachstumsbedingungen oder das Zellwachstum selbst überwacht werden.

Als Reportermoleküle kommen grundsätzlich alle im Stand der Technik, insbesondere für ähnliche Zwecke, bekannten Reportermoleküle in Frage.

Typischerweise sind die Reportermoleküle Farbstoffe oder Fluoreszenzmarker. Einige nicht beschränkende Beispiele sind Fluorescein-Derivate (z.B. 2",7"-Bis-(2-carboxyethyl)-5-(und 6-)carboxyfluorescein (BCECF)), Fluorecin-Derivate, Benzoxanthen-Derivate, Ruthenium-Komplexe etc.

In spezielleren Ausführungsformen sind die Reportermoleküle viskositätssensitiv, pH-sensitiv, CO₂- oder O₂-sensitiv.

Die Reportermoleküle können unter anderem eine Änderung von mindestens einem Parameter in der Kapsel, z.B. eine pH-Änderung oder eine Änderung des Sauerstoffpartialdrucks, durch eine korrespondierende nachweisbare Eigenschaftsänderung der Reportermoleküle in der Kapsel, z.B. eine Farbänderung, eine Änderung der Fluoreszenzemissions- oder -absorptionswellenlänge, eine Änderung der Fluoreszenzlebensdauer, anzeigen.

Typischerweise erfolgt der Nachweis der Änderung des Parameters durch ein spektroskopisches oder spektrometrisches Verfahren, insbesondere ausgewählt aus der Gruppe aus VIS-Spektroskopie, Fluoreszenzspektroskopie, zeitaufgelöste Fluoreszenzspektroskopie, FRET-Spektroskopie etc.

Der Nachweis von pH-Änderungen kann direkt durch Fluoreszenzmessungen erfolgen. Bekannte Fluorophore, um den pH zu messen, sind Fluorescein und Derivate, bzw. HTPS (8-Hydroxypyren-1,3,6,-trisulfonsäure). Dabei verschieben sich Absorptions-und Emissionswellenlänge, was bei fixen Anregungs-/Emissionswellenlängen zu einer Zunahme bzw. Abnahme der emittierten Intensität führt.

Bei der O₂-Messung kann man sich den Effekt zunutze machen, dass gelöster Sauerstoff die Fluoreszenz von Farbstoffen quencht, d.h. die Lebensdauer des angeregten Zustandes beträchtlich verkürzt. Folglich misst man hier nicht die gemittelte Intensität (wie beim pH), sondern zeitaufgelöst den Fluoreszenzabfall nach kurzer Anregung (Anregungs-Blitz). Gut eignen sich Ru-Komplexe (z.B. Ruthenium(II)(4,7-diphenyl-1,10-phenanthrolin)3). In dem Fall würde man den Liganden an die Polymer-Matrix binden und das Ru-Salz dazu geben.

CO₂-Sensoren funktionieren nach demselben Prinzip, mit dem Unterschied, dass die Fluoreszenz-Lebenszeit in der Regel indirekt über einen Fluoreszenz-Transfer (FRET) gemessen wird. Dabei macht man sich zunutze, dass die emittierte Strahlung nur von einem anderen (in der Nähe befindlichen) Fluorophor aufgenommen werden kann, wenn die Lebenszeit des ersten Fluorophors (idR wiederum Ru-Porphyrin-Komplexe) lang genug ist. Man misst dann die Fluoreszenzintensität des zweiten Fluorophors.

Für die Viskositätsmessung nutzt man auch das Stoss-Quenching eines angeregten Zustandes (Stern-Vollmer-Gleichung). Dazu kann im Prinzip jedes Fluorophor verwendet werden, von dem die initiale Abklingzeit der Fluoreszenz gemessen wird (im nsec-Bereich). Durch Viskositätsmessungen kann z.B. der Abbaugrad der Kapseln nachgewiesen bzw. verfolgt werden.

Im Folgenden wird ein im obigen Verfahren verwendeter und hergestellter Zellkultur-Träger in Kapselform beschrieben, der selbst nicht Teil der Erfindung ist.

Dieser Zellkultur-Träger umfasst ein vernetztes biokompatibles Material in Kapselform, welches darin inkorporierte adhärente Zellen aufweist, und ist dadurch gekennzeichnet, dass das vernetzte biokompatible Kapselmaterial durch einen physikalischen oder chemischen Stimulus aufgelöst und die Zellen dadurch freigesetzt werden können.

Spezieller ist der erfindungsgemäß verwendete Zellkultur-Träger dadurch gekennzeichnet, dass die Kapseln durch einen Stimulus, der aus der Gruppe ausgewählt ist, welche eine Temperaturänderung, Druckänderung, Ultraschall, elektromagnetische Strahlung, insbesondere sichtbares Licht und UV, ein pH-Änderung, die Einwirkung von Enzymen, insbesondere Proteasen und zucker-spaltenden Enzymen, Komplexbildnern oder Lösungsmitteln, oder Kombinationen davon umfasst, aufgelöst werden können.

Das vernetzte biokompatible Material des Zellkultur-Trägers ist ein Hydrogel, welches ein protein-basiertes Hydrogel, ein zucker-basiertes Hydrogel, enthaltend modifizierte Oligo- oder Polysaccharide, oder ein synthe-tisches Hydrogel auf der Basis von Polyestern, Polyethern oder Polyalkoholen sein kann und/oder ein thermoresponsives Material, z.B. Poly-NIPAM oder Poly(hydroxypropylcellulose, umfasst.

In speziellen Ausführungsformen umfassen die Kapseln aus vernetztem biokompatiblen Material ein thermoresponsives Material, z.B. Poly-NIPAM oder Poly(hydroxypropylcellulose), einen Polyester, Polyether oder Polyalkohol, Seide, Fibrin oder vernetztes Alginat.

Noch spezieller ist der Zellkultur-Träger dadurch gekennzeichnet, dass das vernetzte Alginat eine Alginat-Gerüststruktur mit angekoppelten Gelatinemolekülen umfasst, vorzugsweise eine Alginatgerüststruktur, bei der jedes zweite bis dreißigste, bevorzugter jedes fünfte bis fünfzehnte, besonders bevorzugt etwa jedes zehnte Zuckermolekül, mit einer Gelatineprotein-Seitenkette versehen ist.

Die Größe der Kapseln ist nicht besonders beschränkt. In der Regel haben die Kapseln eine Größe im Bereich von 0,1 mm bis 40 mm, typischerweise 3 bis 5 mm.

In einer bevorzugten Ausführungsform enthalten die Kapseln aus vernetztem biokompatiblen Material ferner immobilisierte Nährstoffe und/oder Wachstumshormone.

In einer besonders bevorzugten Ausführungsform der Erfindung umfassen die Kapseln aus vernetztem biokompatiblen Material ferner immobilisierte Reportermoleküle wie oben beschrieben. Diese Reportermoleküle können beispielsweise viskositätssensitiv, pH-sensitiv, CO₂- oder O₂-sensitiv sein.

In einer speziellen Ausführungsform sind die Reportermoleküle Farbstoffe oder Fluoreszenzmarker.

Die Verwendung der erfindungsgemäß verwendeten kapselförmigen Zellkultur-Träger mit inkorporierten Reportermolekülen erlaubt beispielsweise den Nachweis einer Änderung von mindestens einem Reaktionsparameter in der Kapsel, z.B. eine pH-Änderung oder eine Änderung des Sauerstoffpartialdrucks, durch eine korrespondierende nachweisbare Eigenschaftsänderung der Reportermoleküle, z.B. eine Farbänderung, eine Änderung der Fluoreszenzemissions- oder -absorptionswellenlänge, eine Änderung der Fluoreszenzlebensdauer.

Der Nachweis der Änderung des Reaktionsparameters kann beispielsweise durch ein spektroskopisches oder spektrometrisches Verfahren, insbesondere ausgewählt aus der Gruppe aus VIS-Spektroskopie, Fluoreszenzspektroskopie, zeitaufgelöste Fluoreszenzspektroskopie, FRET-Spektroskopie etc., erfolgen.

Der erfindungsgemäß verwendete Zellkultur-Träger eignet sich für eine Vielfalt von Einsatzgebieten, beispielsweise präparative Zellkulturtechniken, einschließlich Bioreaktoren zur Gewinnung bestimmter Biomoleküle, und analytische Zellkulturtechniken, z.B. in der medizinischen und pharmazeutischen Analytik.

Dementsprechend kann ein Zellkultur-Träger wie oben beschrieben insbesondere in einem Reaktor für die Zellkultur oder einer Vorrichtung für die Analytik eingesetzt werden.

### Vorteile der Erfindung:

Erstens kann der Reaktor auch für sehr kleine Start-Zellzahlen bereits in seiner Endgröße (Ziel-Zellzahl) betrieben werden. Dadurch, dass die Zellen in einer lokalen Mikroumgebung eingeschlossen sind, hat das Reaktorvolumen als solches weniger Bedeutung und die Zellen können lokal über Cytokine/Wachstumsfaktoren etc. miteinander kommunizieren, ohne dass die Konzentration dieser Stoffe im Reaktor homogen vorhanden sein muss.

Zweitens werden im gesamten Vermehrungsprozess nur Flüssigkeiten und Suspensionen gehandhabt. Es werden lediglich herkömmliche Pump- und Filteranlagen benötigt. Die Förderung und Prozessierung von Festkörpern entfällt völlig.

Drittens wachsen die Zellen nicht allein auf der Oberfläche der Kapseln sondern mehrheitlich in ihrem Innern. Dadurch wird das Reaktorvolumen bei hoher Volumenbelegung wesentlich effizienter genutzt.

Viertens sind die Zellen im Innern der Kapseln weitgehend mechanisch geschützt. Scher- und Prallkräfte, wie sie beim Umwälzen des Mediums unvermeidlich auftreten, können viel weniger Schaden anrichten als in herkömmlicher Carrier-Kultur. Gleichzeitig sind höhere Strömungsgeschwindigkeiten bzw. Volumenströme möglich.

Fünftens ist die Beobachtung/Analyse der Zellen in der Kapsel einfacher als auf Mikrocarriern. Die Kapseln sind in Regel transparent und können vergleichsweise leicht mikroskopiert werden. Gleichzeitig ist es, wie oben geschildert, möglich, Sonden- oder Reportermoleküle im Gel der Kapseln zu immobilisieren und optisch auszulesen. Beispiele für solche Moleküle sind pH-abhängige Fluoreszenzfarbstoffe oder Farbstoffe, bei denen die Lebensdauer des angeregten Zustands von der Sauerstoffkonzentration abhängt (pO₂-Messung durch Fluoreszenz-Quenching). Solche Reportermoleküle können leicht optisch ausgelesen werden und zeigen den Versorgungszustand der verkapselten Zellen direkt an.

Sechstens kann die Kapsel selbst Nährstoffe und Wachstumshormone enthalten (z.B. immobilisiert), so dass mit einem vergleichsweise mageren Medium gearbeitet werden kann. Durch die lokale Bereitstellung der Nährstoffe/Wachstumhormone kann das Medium wesentlich effizienter genutzt werden.

Siebtens entfallen die herkömmlichen Schritte der Inokulation/Zellernte völlig und werden durch die Prozesse des Verkapselns bzw. Kapselauflösens ersetzt. Dies ermöglicht eine sehr viel einfachere und schonendere Einbringung/Austragung der Zellen in den Reaktor bzw. aus ihm heraus und gestattet einen verlustfreieren Umgang mit den Zellen.

### Kurzbeschreibung der Figuren

**Fig. 1** zeigt schematisch die Hauptschritte des erfindungsgemäßen Kultivierungsverfahrens unter Verwendung eines kapselförmigen Zellkultur-Trägers. 1: Suspendieren von Zellen in einem Medium; 2: Vertropfen der Zellsuspension und Kapselbildung; 3: Expansionsphase; 4. Auflösen der Kapseln; 5: Überführen der freigesetzten Zellen in den nächsten Zyklus
**Fig. 2** zeigt beispielhaft die Entwicklung der Zellzahlen bei Kultivierung in Hydrogelgelkapseln.
**Fig. 3** zeigt Zellen/Kapseln in verschiedenen Stadien der Kultivierung.

Das folgende Ausführungsbeispiel dient zur näheren Erläuterung der Erfindung, ohne dieselbe jedoch auf die speziellen Parameter und Bedingungen der Beispiele zu beschränken.

### BEISPIEL 1

### Kultivierung von Zellen in Alginat-Gelatine-Kapseln

Das Kapselmaterial wurde durch kovalente chemische Verbindung von Alginat mit Gelatine hergestellt. Das Alginat bildet hierbei das eigentliche Hydrogel-Gerüst (Backbone), an dem durch die Gelatine Bindungsstellen für die Zellen angefügt werden. In reinen Alginat-Kapseln können Zellen nicht adhärieren/proliferieren. Auch in physikalischen Alginat/- Gelatine-Mischungen funktioniert die Vermehrung von adhärent wachsenden Zellen nicht. Deshalb ist eine chemische Kopplung notwendig.

Die Kopplungsreaktion erfolgte mittels Carbodiimid:
In einem Ansatz wurden 40 mg Natriumalginat im 5 ml MES-Puffer (0,2 M, MES, 0,3 M NaCl, pH = 6,5) bei Raumtemperatur unter Rühren für ca. 30 Minuten gelöst. Gleichzeitig wurden 1,2 g Gelatine (Solugel P/400 von PB Gelatins GmbH, ca. 3 kDa mittlere Molmasse; es handelt sich um Gelatine-Hydrolysat (d.h. kleinere Gelatine-Fragmente) in 15 ml MES-Puffer bei 37°C und 200 rpm für ca. 1 h gelöst. Kurz vor Reaktionsbeginn wurden 6,7 µl (0,04 mmol) N-(3-dimethylaminopropyl)-N-ethylcarbodiimid (EDC) in 10 ml MES-Puffer vorgelegt und mit 4,5 mg (0,02 mmol) N-Hydroxysuccinimid-Natriumsalz (Sulfo-NHS) versetzt. Bei 0°C wurde anschließend das in MES-Puffer gelöste Alginat zu der Lösung gegeben und 5 min. gerührt. Danach wurde die Gelatine zur Reaktionslösung gegeben und 2 h bei RT gerührt. Die Reinigung erfolgte mittels Dialyse gegen destilliertes Wasser für ca. 5 Tage. Das Material kann zur Lagerung gefriergetrocknet werden.

Das synthetisierte Material wurde in Zellkultur gelöst und durch Eintropfen in Ca²⁺-haltige Lösung zu Kapseln verarbeitet. Vorher in dem Zellkulturmedium suspendierte Zellen sind dann in den Kapseln immobilisiert und können in ihnen adhärent wachsen. Nachdem die Kapseln voll gewachsen sind (je nach Zellsorte und Einsaatdichte ca. 5-20 Tage), wurde das Kapselmaterial durch Zugabe von Citrat oder EDTA aufgelöst und die Zellen freigesetzt.

Die freigesetzten Zellen befinden sich in Suspension und können in dieser abermals mit dem Gelbildner versetzt und verkapselt werden. Dieser Zyklus kann beliebig oft wiederholt werden. Der Vermehrungsfaktor pro Zyklus beträgt etwa 10. Eine Vermehrung um den Faktor 10⁴ benötigt also lediglich eine 4-5-fache Wiederholung des Zyklus.

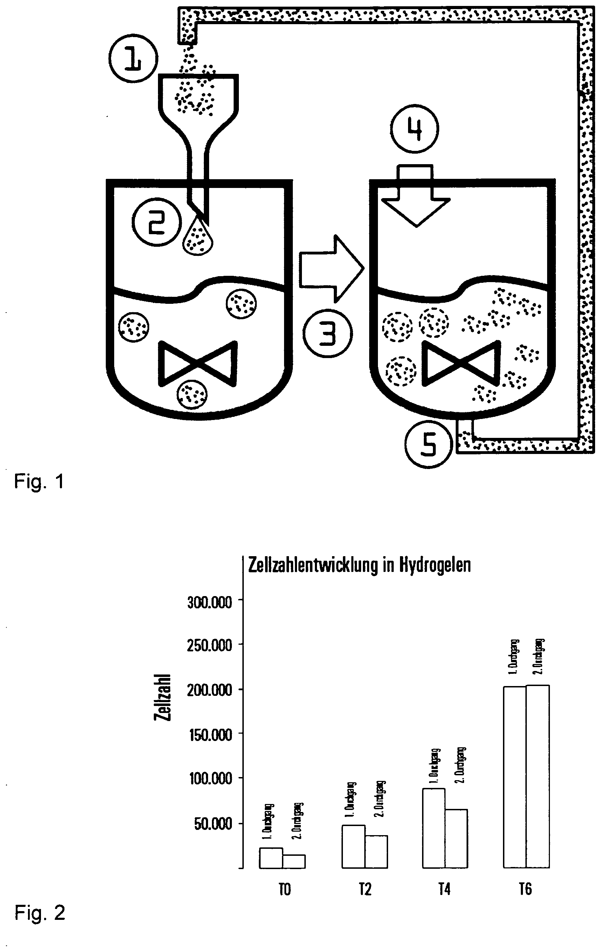

Die oben beschriebenen Hauptschritte können anhand des in Fig. 1 gezeigten Schemas in generalisierter Form zusammengefasst werden. Schritt (1): Zellen werden in Medium suspendiert, welches auch unvernetzte Hydrogel-Matrix enthält (typischerweise 1-4 % m/v); Schritt (2): Die Suspension wird in ein gelbildendes Medium vertropft und bildet hier stabile Kapseln, in denen Zellen wachsen können; Schritt (3): Während der Expansionsphase wird die Kapsel-Suspension gerührt bzw. Medium getauscht etc.; Schritt 4: Sobald die Zellen in den Kapseln konfluent sind (optional Readout durch Farbstoffe) werden die Kapseln durch einen äußeren Stimulus (z.B. Zugabe von Komplexbildnern, pH-Änderung, Temperaturänderung etc.) aufgelöst; Schritt (5): Die freigesetzten Zellen sedimentieren und können für einen nächsten Zyklus mit Medium und unvernetzter Matrix versetzt werden.

Fig. 2 zeigt die Entwicklung der Zellzahl in den wie oben beschrieben hergestellten Hydrogelkapseln. Die Zellen wurden am Tag 0 (T0) verkapselt und wuchsen in den Kapseln bis zum Tag 7 (erster Durchgang, linke Balken). Danach wurden die Kapseln aufgelöst und die freigesetzten Zellen abermals verkapselt und für weitere 7 Tage kultiviert (zweiter Durchgang, rechte Balken). Pro Durchgang/Zyklus ergab sich eine Vermehrung um ca. Faktor 10. Durch zyklisches Wiederholen des Prozesses können Vermehrungsraten >> 10³ erzielt werden.

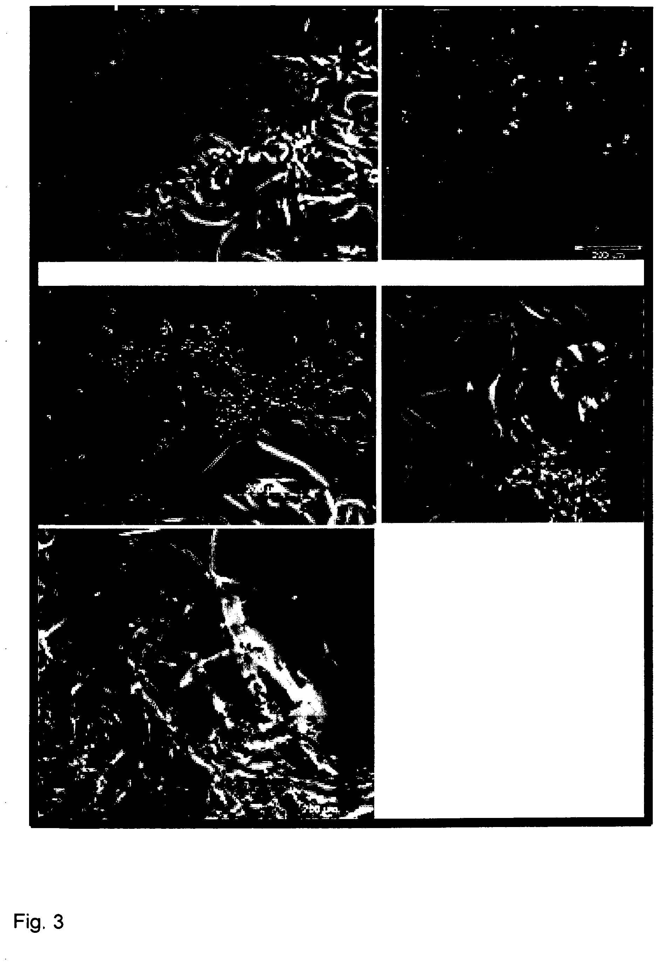

Fig. 3 zeigt Zellen/Kapseln in verschiedenen Stadien der Kultivierung.

Die obere Reihe zeigt die Zellen in der Kapsel kurz nach Verkapselung. Links ist eine lichtmikroskopische Aufnahme, rechts eine Vitalfärbung (Kerne: Hoechst, Zellplasma: Fluorescein-Diacetat).

Die mittlere Reihe zeigt die Zellen während der Expansion. Rechts, in der Vitalfärbung, ist deutlich zu sehen, dass die Zellen im Gel adhärent werden (ausgestreckte Morphologie).

Das untere Bild zeigt den Prozess der Kapseldegradierung bzw. Freisetzung der Zellen.

## Patentansprüche

1. Verfahren zur Kultivierung von Zellen in Adhäsionskultur, umfassend mindestens die folgenden Schritte:
a) Lösen oder Suspendieren eines vernetzungsfähigen, biokompatiblen Materials mit Adhäsionsstellen für Zellen in einem Zellkulturmedium,
b) Suspendieren von Zellen in dem Zellkulturmedium, welches das vernetzungsfähige, biokompatible Material enthält, oder in einem Medium, das mindestens eine Komponente enthält, welche zur Vernetzung des vernetzungsfähigen, biokompatiblen Materials erforderlich ist,
c) Eintropfen der Zellsuspension in ein Medium unter Bedingungen, welche die Vernetzung des biokompatiblen Materials initiieren oder erlauben, wobei entweder die Zellsuspension oder das Medium, in welches die Zellsuspension eingetropft wird, das vernetzungsfähige biokompatible Material enthält, und wobei die Bedingungen, welche die Vernetzung des biokompatiblen Materials in Schritt c) initiieren oder erlauben, die Exposition des biokompatiblen Materials gegenüber einem physikalischen oder chemischen Stimulus umfassen, welcher aus der Gruppe, die eine Temperaturänderung, Druckänderung, Ultraschall, elektromagnetische Strahlung, insbesondere sichtbares Licht und UV, ein pH-Änderung, die Einwirkung von Enzymen, Radikalstartern, Wasserzugabe oder Wasserausschluss, Ionen, insbesondere Kationen, umfasst, ausgewählt ist, und
d) Bilden von stabilen, vorzugsweise porösen, Kapseln aus vernetztem biokompatiblem Material, welche inkorporierte adhärente Zellen enthalten,
e) Proliferieren der adhärenten Zellen in den Kapseln für eine vorgegebene Zeitspanne,
f) Auflösen des Kapselmaterials durch einen physikalischen oder chemischen Stimulus und Freisetzen der Zellen als Zellsuspension, wobei der Stimulus aus der Gruppe ausgewählt ist, welche eine Temperaturänderung, Druckänderung, Ultraschall, elektromagnetische Strahlung, insbesondere sichtbares Licht und UV, ein pH-Änderung, die Einwirkung von Enzymen, insbesondere Proteasen und zuckerspaltenden Enzymen, Komplexbildnern oder Lösungsmitteln, oder Kombinationen davon umfasst,
**dadurch gekennzeichnet, dass** die in Schritt f) freigesetzten Zellen erneut in einem Zellkulturmedium, welches ein vernetzungsfähiges, biokompatibles Material enthält, oder in einem Medium, das mindestens eine Komponente enthält, welche zur Vernetzung des vernetzungsfähigen, biokompatiblen Materials erforderlich ist, suspendiert und die Schritte c)-f) mindestens einmal wiederholt werden,
und wobei das vernetzungsfähige biokompatible Material ein Material darstellt oder umfasst, welches ein Hydrogel bilden kann, wobei das Hydrogel ein protein-basiertes Hydrogel, ein zucker-basiertes Hydrogel, enthaltend modifizierte Oligo- oder Polysaccharide, oder ein synthetisches Hydrogel auf der Basis von Polyestern, Polyethern oder Polyalkoholen ist und/oder ein thermoresponsives Material, z.B. Poly-NIPAM oder Poly(hydroxypropylcellulose), umfasst.

2. Verfahren nach Anspruch 1, **dadurch gekennzeichnet, dass** Schritt c) unter Bedingungen durchgeführt wird, welche die Porenbildung im Kapselmaterial fördern, insbesondere **dadurch gekennzeichnet, dass** in das Medium, in welches die Zellsuspension eingetropft wird, ein Gas eingepresst wird oder ein gasbildendes partikuläres Material, z.B. ein Carbonat, welches nach Exposition gegenüber einem physikalischen oder chemischen Stimulus, z.B. Säure, Gas freisetzt, in das Kapselmaterial eingeschlossen wird.

3. Verfahren nach Anspruch 1, **dadurch gekennzeichnet, dass** der physikalische Stimulus in Schritt c) eine Ultraschallbehandlung des biokompatiblen Materials ist und der chemische Stimulus in Schritt c) der Kontakt des biokompatiblen Materials mit einem chemischen Agens, insbesondere ausgewählt aus der Gruppe aus einem Enzym, z.B. Thrombin, und einem 2-wertigen Kation, z.B. Ca²⁺ oder Ba²⁺, ist oder diesen umfasst.

4. Verfahren nach Anspruch 1, **dadurch gekennzeichnet, dass** der Stimulus in Schritt f) die Einwirkung von Proteasen, die von den adhärenten Zellen selbst produziert werden, darstellt oder umfasst.

5. Verfahren nach einem der Ansprüche 1 bis 4, **dadurch gekennzeichnet, dass** das vernetzungsfähige, biokompatible Material durch eine chemische Kopplungsreaktion mit Adhäsionsstellen für Zellen versehen wurde,
wobei die Adhäsionsstellen für Zellen insbesondere spezifische oder unspezifische Adhäsions- oder Bindungsmotive für biologische Zellen, z.B. Aminogruppen, RGD-Peptide, RAD-Peptide oder Analoga davon, darstellen oder umfassen.

6. Verfahren nach einem der Ansprüche 1 bis 5, **dadurch gekennzeichnet, dass** die Kapseln aus vernetztem biokompatiblen Material ein thermoresponsives Material, z.B. Poly-NIPAM oder Poly(hydroxypropylcellulose), einen Polyester, Polyether oder Polyalkohol, Seide, Fibrin oder vernetztes Alginat umfassen.

7. Verfahren nach einem der Ansprüche 1 bis 6, **dadurch gekennzeichnet, dass** an eine Alginat-Gerüststruktur Gelatinemoleküle angekoppelt wurden, vorzugsweise mittels einer Carbodiimid-Kopplungsreaktion, und die Vernetzung dieses Alginat-Gelatine-Vorläufermaterials durch Kontaktierung mit einem zweiwertigen Kation, insbesondere Ca²⁺ oder Ba²⁺, erfolgt.

8. Verfahren nach einem der Ansprüche 1-7, **dadurch gekennzeichnet, dass** die Kapseln aus vernetztem biokompatiblen Material ferner immobilisierte Nährstoffe/Wachstumshormone und/oder Reportermoleküle enthalten, wobei die Reportermoleküle insbesondere pH-sensitiv, viskositätssensitiv, CO₂- oder O₂-sensitiv sind und/oder Farbstoffe oder Fluoreszenzmarker sind.

9. Verfahren nach Anspruch 8, **dadurch gekennzeichnet, dass** die Reportermoleküle eine Änderung von mindestens einem Parameter in der Kapsel, z.B. eine pH-Änderung oder eine Änderung des Sauerstoffpartialdrucks, durch eine korrespondierende nachweisbare Eigenschaftsänderung der Reportermoleküle, z.B. eine Farbänderung, eine Änderung der Fluoreszenzemissions- oder -absorptionswellenlänge, eine Änderung der Fluoreszenzlebensdauer, anzeigen.

10. Verfahren nach Anspruch 9, **dadurch gekennzeichnet, dass** der Nachweis der Änderung des Parameters durch ein spektroskopisches oder spektrometrisches Verfahren, insbesondere ausgewählt aus der Gruppe aus VIS-Spektroskopie, Fluoreszenzspektroskopie, zeitaufgelöste Fluoreszenzspektroskopie, FRET-Spektroskopie etc., erfolgt.

## Claims

1. A method for cultivating cells in adhesion culture, comprising at least the following steps:
a) dissolving or suspending a cross-linkable, biocompatible material with adhesion sites for cells in a cell culture medium,
b) suspending cells in the cell culture medium which contains the cross-linkable, biocompatible material, or in a medium which contains at least one component which is required to cross-link the cross-linkable, biocompatible material,
c) introducing the cell suspension in drops into a medium under conditions which initiate or allow the cross-linking of the biocompatible material, wherein either the cell suspension or the medium, into which the cell suspension is introduced in drops, contains the cross-linkable, biocompatible material, and wherein the conditions which initiate or allow the cross-linking of the biocompatible material in step c) encompass exposing the biocompatible material to a physical or chemical stimulus which is selected from the group comprising a temperature change, pressure change, ultrasound, electromagnetic radiation, in particular visible light and UV, a pH change, the action of enzymes, radical starters, addition of water or exclusion of water, ions, in particular cations, and
d) forming stable, preferably porous capsules of cross-linked, biocompatible material which contain incorporated, adherent cells,
e) proliferating the adherent cells in the capsules for a defined time period,
f) dissolving the capsule material by means of a physical or chemical stimulus and releasing the cells as a cell suspension, wherein the stimulus is selected from the group comprising temperature change, pressure change, ultrasound, electromagnetic radiation, in particular visible light and UV, a pH change, the action of enzymes, in particular proteases and sucroclastic enzymes, complexing agents or solvents, or combinations thereof, **characterised in that** the cells released in step f) are once again suspended in a cell culture medium, which contains a cross-linkable, biocompatible material, or in a medium which contains at least one component which is required to cross-link the cross-linkable, biocompatible material and steps c)-f) are repeated at least once, and wherein the cross-linkable biocompatible material represents or comprises a material which is capable to form a hydrogel, wherein the hydrogel is a protein-based hydrogel, a sugar-based hydrogel, containing modified oligo- or polysaccharides, or a synthetic hydrogel on the basis of polyesters, polyethers or polyalcohols, and/or comprises a thermoresponsive material, e.g. poly-NIPAM or poly(hydroxypropyl)cellulose.

2. The method according to Claim 1, **characterised in that** step c) is carried out under conditions which support pore formation in the capsule material, in particular **characterised in that** a gas is injected into the medium into which the cell suspension is introduced in drops or a gas-forming particulate material, e.g. a carbonate, which releases gas after exposure to a physical or chemical stimulus, e.g. acid, is incorporated in the capsule material.

3. The method according to Claim 1, **characterised in that** the physical stimulus in step c) is an ultrasound treatment of the biocompatible material and the chemical stimulus in step c) is the contact of the biocompatible material with a chemical agent, in particular selected from the group of an enzyme, e.g. thrombin, and a bivalent cation, e.g. Ca²⁺ or Ba²⁺, or comprises said contact.

4. The method according to Claim 1, **characterised in that** the stimulus in step f) represents or comprises the action of proteases which are produced by the adherent cells themselves.

5. The method according to any one of Claims 1 to 4, **characterised in that** the cross-linkable, biocompatible material was provided with adhesion sites for cells by a chemical coupling reaction, wherein the adhesion sites for cells in particular represent or comprise specific or non-specific adhesion or binding motifs for biological cells, e.g. amino groups, RGD peptides, RAD peptides or analogues thereof.

6. The method according to any one of Claims 1 to 5, **characterised in that** the capsules composed of cross-linked, biocompatible material comprise a thermoresponsive material, e.g. poly-NIPAM or poly(hydroxypropyl)cellulose, a polyester, polyether or polyalcohol, silk, fibrin or cross-linked alginate.

7. The method according to any one of Claims 1 to 6, **characterised in that** gelatine molecules are coupled to an alginate framework structure, preferably by means of a carbodiimide coupling reaction, and the cross-linking of this alginate-gelatine precursor material is effected by contacting with a bivalent cation, in particular Ca²⁺ or Ba²⁺.

8. The method according to any one of Claims 1 to 7, **characterised in that** the capsules composed of cross-linked, biocompatible material further contain immobilised nutrients/growth hormones and/or reporter molecules, wherein the reporter molecules are in particular pH-sensitive, viscosity-sensitive, CO₂- or O₂-sensitive and/or are dyes or fluorescence markers.

9. The method according to Claim 8, **characterised in that** the reporter molecules display a change of at least one parameter in the capsule, e.g. a pH change or a change in the oxygen partial pressure, by a corresponding verifiable change in properties of the reporter molecules, e.g. a colour change, a change in fluorescence emission or absorption wavelength, a change in fluorescence lifetime.

10. The method according to Claim 9, **characterised in that** verifying the change of the parameter is effected by a spectroscopic or spectrometric method, in particular selected from the group of VIS spectroscopy, fluorescence spectroscopy, time-resolved fluorescence spectroscopy, FRET spectroscopy, etc.

## Revendications

1. Procédé pour la culture cellulaire selon la méthode de la goutte pendante, comprenant au moins les étapes suivantes :
a) le détachement ou la suspension d'un matériau biocompatible, réticulable avec des points d'adhésion pour des cellules dans un milieu de culture cellulaire,
b) la suspension de cellules dans le milieu de culture cellulaire, qui contient le matériau biocompatible, réticulable, ou dans un milieu qui contient au moins un composant, lequel est nécessaire à la réticulation du matériau biocompatible, réticulable,
c) l'introduction par goutte-à-goutte de la suspension de cellules dans un milieu dans des conditions qui amorcent ou permettent la réticulation du matériau biocompatible, dans lequel soit la suspension de cellules, soit le milieu dans lequel la suspension de cellules est introduite par goutte-à-goutte, contient le matériau biocompatible, réticulable, et dans lequel les conditions qui amorcent ou permettent la réticulation du matériau biocompatible à l'étape c), comportent l'exposition du matériau biocompatible à un stimulus physique ou chimique qui est sélectionné à partir du groupe qui comporte une modification de température, une modification de pression, un ultrason, un rayonnement électromagnétique, en particulier une lumière visible et UV, une modification de pH, l'action d'enzymes, des amorceurs de radicaux, une addition d'eau ou une exclusion d'eau, des ions, en particulier des cations, et
d) la formation de capsules stables, de préférence poreuses, en matériau biocompatible réticulé qui contiennent des cellules adhérentes incorporées,
e) la prolifération des cellules adhérentes dans les capsules pour un intervalle prédéfini,
f) la dissolution du matériau de capsule par un stimulus physique ou chimique et la libération des cellules comme suspension de cellules, dans lequel le stimulus est sélectionné à partir du groupe qui comporte une modification de température, une modification de pression, un ultrason, un rayonnement électromagnétique, en particulier une lumière visible et UV, une modification de pH, l'action d'enzymes, en particulier de protéases et d'enzymes dégradant le sucre, des agents complexants ou des solvants, ou des combinaisons de ceux-ci, **caractérisé en ce que** les cellules libérées à l'étape f) sont suspendues de nouveau dans un milieu de culture cellulaire qui contient un matériau biocompatible, réticulable, ou dans un milieu qui contient au moins un composant, lequel est nécessaire à la réticulation du matériau biocompatible, réticulable, et les étapes c)-f) sont répétées au moins une fois,
et dans lequel le matériau biocompatible, réticulable constitue ou comporte un matériau qui peut former un hydrogel, dans lequel l'hydrogel est un hydrogel à base de protéine, un hydrogel à base de sucre, contenant des oligo- ou polysaccharides modifiés, ou un hydrogel synthétique sur la base de polyesters, polyéthers ou polyalcools et/ou un matériau thermorépondeur, par exemple le poly-NIPAM ou la poly(hydroxypropylcellulose).

2. Procédé selon la revendication 1, **caractérisé en ce que** l'étape c) est réalisée dans des conditions qui favorisent la formation de pores dans le matériau de capsule, en particulier **caractérisé en ce que** dans le milieu dans lequel la suspension de cellule est introduite par goutte-à-goutte, un gaz est injecté ou un matériau particulaire formant du gaz, par exemple un carbonate qui libère, après l'exposition par rapport à un stimulus chimique ou physique, par exemple de l'acide, du gaz, est enfermé dans le matériau de capsule.

3. Procédé selon la revendication 1, **caractérisé en ce que** le stimulus physique à l'étape c) est un traitement par ultrason du matériau biocompatible et le stimulus chimique à l'étape c) est le contact du matériau biocompatible avec un agent chimique, en particulier sélectionné à partir du groupe constitué d'une enzyme, par exemple la thrombine, et d'un cation à deux valeurs, par exemple Ca²⁺ ou Ba²⁺ ou comporte celui-ci.

4. Procédé selon la revendication 1, **caractérisé en ce que** le stimulus à l'étape f) constitue ou comporte l'action de protéases, qui sont produites par les cellules adhérentes elles-mêmes.

5. Procédé selon l'une des revendications 1 à 4, **caractérisé en ce que** le matériau biocompatible, réticulable a été pourvu par une réaction de couplage chimique d'endroits d'adhésion pour des cellules, dans lequel les endroits d'adhésion pour des cellules constituent ou comportent en particulier des motifs d'adhésion ou de liaison spécifiques ou non spécifiques pour des cellules biologiques, par exemple des groupes amino, des peptides RGD, des peptides RAD ou des analogues de ceux-ci.

6. Procédé selon l'une des revendications 1 à 5, **caractérisé en ce que** les capsules en matériau biocompatible, réticulé comportent un matériau thermorépondeur, par exemple le poly-NIPAM ou la poly(hydroxypropylcellulose), un polyester, un polyéther ou un polyalcool, la soie, la fibrine ou un alginate réticulé.

7. Procédé selon l'une des revendications 1 à 6, **caractérisé en ce que** des molécules de gélatine ont été couplées à une structure cadre d'alginate, de préférence au moyen d'une réaction de couplage de carbodiimide, et la réticulation de ce matériau précurseur d'alginate et de gélatine est effectuée par mise en contact avec un cation à deux valeurs, en particulier Ca²⁺ ou Ba²⁺.

8. Procédé selon l'une des revendications 1 à 7, **caractérisé en ce que** les capsules en matériau biocompatible réticulé contiennent de plus des nutriments/hormones de croissance immobilisé(es) et/ou des molécules rapporteuses, dans lequel les molécules rapporteuses sont en particulier sensibles au pH, sensibles à la viscosité, sensibles au CO₂ ou à l'O₂ et/ou sont des colorants ou des marqueurs de fluorescence.

9. Procédé selon la revendication 8, **caractérisé en ce que** les molécules rapporteur indiquent une modification d'au moins un paramètre dans la capsule, par exemple une modification de pH ou une modification de la pression partielle d'oxygène, par une modification de propriété détectable correspondante des molécules rapporteuses, par exemple une modification de couleur, une modification de la longueur d'émission de fluorescence ou d'onde d'absorption de fluorescence, une modification de la durée de vie de fluorescence.

10. Procédé selon la revendication 9, **caractérisé en ce que** la détection de la modification du paramètre est effectuée par un procédé spectroscopique ou spectrométrique, en particulier sélectionné à partir du groupe constitué de la spectroscopie VIS, la spectroscopie de fluorescence, la spectroscopie de fluorescence résolue dans le temps, la spectroscopie FRET, etc.
